# EUROPÄISCHE PATENTANMELDUNG

(11) **EP 0 578 874 A1**
(43) Veröffentlichungstag der Anmeldung: **19.01.1994**
(21) Anmeldenummer: 92250188.7
(22) Anmeldetag: 16.07.1992
(51) Int. Cl.: A61M 13/00, G05D 16/06

(54) **Gasanschlussvorrichtung für Insufflationsgeräte**

(71) Anmelder: Wiest, Peter P., Dipl.-Ing., D-14052 Berlin (DE)
(72) Erfinder: Wiest, Peter P., Dipl.-Ing., D-14052 Berlin (DE)
(74) Vertreter: Lüke, Dierck-Wilm, Dipl.-Ing.

(57) **Zusammenfassung**

Eine Gasanschlußvorrichtung für Insufflationsgeräte, insbesondere zur miniinvasiven Chirurgie sowie zur Laparoskopie und Hysteroskopie, umfaßt einen Anschlußstutzen 3 für die Gaszufuhr, einen Druckminderer 4, ein Sicherheitsventil 5, einen, mit einem Magnetventil 6 versehenen Gasauslaßstutzen 7 und ein Druckanzeigegerät 8.

Um alle Bauteile der Gasanschlußvorrichtung montagefreundlich und mit geringstem Aufwand an Bauteilen vormontiert in ein Insufflationsgerät einbauen zu können, ist erfindungsgemäß vorgesehen, daß der Anschlußstutzen 3, der Druckminderer 4, das Sicherheitsventil 5 das Magnetventil 6 mit Gasauslaßstutzen 7 und das Druckmeßgerät 8 an einen gemeinsamen metallischen Hauptkörper 1 angeschlossen und durch im Hauptkörper 1 ausgebildete Kanäle 10, 22, 23, 44 miteinander verbunden sind. Dabei ergibt sich gleichzeitig durch den lösbaren und auswechselbaren Einbau des Anschlußstutzens 3 in den Hauptkörper 1 die Möglichkeit, nach der Endmontage des Insufflationsgerätes eine Anpassung des Anschlußstutzens 3 an die Erfordernisse des Gasflaschenanschlusses vorzunehmen.

## Beschreibung

Die Erfindung bezieht sich auf eine Gasanschlußvorrichtung für Insufflationsgeräte gemäß den Merkmalen des Oberbegriffes des Anspruches 1. Die Gasanschlußvorrichtung dient zur Verbindung eines medizinischen Insufflationsgerätes, insbesondere für die miniinvasive Chirurgie sowie für die Laparoskopie und die Hysteroskopie, mit einer Gasvorratsflasche und zur Minderung des Gasflaschendruckes auf den Zulässigen Arbeitsdruck des Insufflationsgerätes (z.B. 50 mmHg) sowie zur Überwachung, Messung und Steuerung des den Gasauslaßstutzen der Gasanschlußvorrichtung verlassenden Arbeitsdruckes des Insufflationsgases, insbesondere CO₂.

Bei einer bisher bekannten Ausführungsform der Gasanschlußvorrichtung eines herkömmlichen Insufflationsgerätes ist der Anschlußstutzen in der Gerätewandung montiert und über ein Kupferrohr mit dem Druckminderer verbunden. Dieser ist über einen Preßluftschlauch mit dem Sicherheitsventil und über ein weiteres Kupferrohr mit einem druckanzeigenden Manometer verbunden. Schließlich ist ein weiterer Preßluftschlauch zwischen dem Sicherheitsventil und dem Magnetventil vorhanden. Die Kupferrohre und Preßluftschläuche sind über Verschraubungen mit speziellen Überwurfmuttern mit den jeweiligen Bauteilen der Gasanschlußvorrichtung verbunden. Somit sind bei der Montage der vorbekannten Gasanschlußvorrichtung innerhalb des Gehäuses eines Insufflationsgerätes mehrere Einzelbaugruppen einzubauen und über Kupferrohre und Preßluftschläuche miteinander zu verbinden. Die Verarbeitung der Kupferrohre durch Zuschneiden, Biegen, Verchromen, späneentfernung od.dgl. erfordert einen erheblichen Arbeitsaufwand. Außerdem entstehen erhebliche Kosten in der Lagerhaltung sowie bei der Beschaffung der Kupferrohre, Preßluftschläuche, Verschraubungen und Überwurfmuttern. Insgesamt ergeben sich relativ hohe Montagezeiten und eine große Anzahl von Fehlerstellen.

Der Erfindung liegt von daher die Aufgabe zugrunde, eine Gasanschlußvorrichtung für Insufflationsgeräte der gattungsgemässen Art zu schaffen, welche im Hinblick auf kürzere Montagezeiten, Verminderung der Anzahl der Bauteile und Fehlerstellen und geringere Beschaffungs-und Lagerkosten vereinfacht ist.

Zur Lösung dieser Aufgabe sieht die Erfindung vor, daß der Anschlußstutzen, der Druckminderer, das Sicherheitsventil und das Magnetventil mit dem Gasauslaßstutzen an einem gemeinsamen Hauptkörper angeschlossen und durch im Hauptkörper ausgebildete Kanäle miteinander verbunden sind. Der insbesondere aus Metall gebildete Hauptkörper wird vor dem Einbau in das Gehäuse des Insufflationsgerätes vormontiert, wobei der Druckminderer, das Sicherheitsventil und das mit dem Gasauslaßstutzen versehene Magnetventil bereits mit den jeweiligen im Hauptkörper ausgebildeten Kanälen und fest mit dem Hauptkörper verbunden sind. Anschließend wird die derart vormontierte und geprüfte Gasanschlußvorrichtung mit der Gerätewandung des Insufflationsgerätes fest verbunden. Schließlich wird der Anschlußstutzen von außen durch die Gerätewandung hindurch in den Hauptkörper der Gasanschlußvorrichtung eingebracht. Die erfindungsgemäße Gasanschlußvorrichtung benötigt nur etwa die halbe Anzahl an Bauelementen, welche für die herkömmliche Gasanschlußvorrichtung erforderlich sind.

In besonders bevorzugter Weise ist der Anschlußstutzen für die Gaszufuhr lösbar und auswechselbar im Hauptkörper angeordnet. Damit kann der Anschlußstutzen an die unterschiedlichen Bauarten von Stutzen zur Verbindung mit einer Gasvorratsflasche leicht angepaßt werden, wie insbesondere Cartuschen-, Pinn-Index-, amerikanische-CO₂-, deutsche-CO₂-, japanische CO₂ und deutsche N₂0-Stutzen. Dadurch ergibt sich die Möglichkeit des Austausches der verschiedenen Anschlußstutzen für die Gaszufuhr, ohne daß das Insufflationsgerät selbst demontiert werden muß. Ferner ergeben sich durch den Verzicht auf das früher angewendete Verkleben der einzelnen Bauelemente definierte Abdichtungsverhältnisse, wodurch die Servicefreundlichkeit des Insufflationsgerätes verbessert wird. Der Kompaktzusammenbau des Anschlußstutzens für die Gaszufuhr, des Druckminderers, des Sicherheitsventiles und eines Druckwächters in einem einstückigen Hauptkörper und die Verbindung der Bauteile durch im Hauptkörper ausgebildete Kanäle ermöglicht den Verzicht auf Kupferrohrverbindungen und Preßluftschläuche zwischen den einzelnen Bauelementen. Dadurch werden im Hinblick auf eine Qualitätsverbesserung Fehlerquellen ausgeschlossen und erhebliche Ersparnisse an Kupferrohr und dessen Verarbeitung,wie Zuschneiden, Biegen, Verchromen, Späneentfernung usw., ermöglicht. Weitere Ersparnisse ergeben sich durch den Verzicht auf Verschraubungen mit entsprechenden Überwurfmuttern, wodurch Kosten in der Lagerhaltung sowie bei der Beschaffung- und Teileverwaltung eingespart werden können. Schließlich wird auf eine zusätzliche Gasanschluß-Verdrehsicherung verzichtet. Es ergeben sich erheblich kürzere Montagezeiten sowie Prüfungsmöglichkeiten der Gasanschlußvorrichtung ( Gasanschlußkombination) vor deren Einbau in das Insufflationsgerät.

Weitere vorteilhafte Ausgestaltungen der Erfindung ergeben sich aus den Unteransprüchen.

Die erfindungsgemäße Gasanschlußvorrichtung für Insufflationsgeräte ist nachfolgend anhand eines in den Zeichnungen dargestellten Ausführungsbeispieles näher erläutert. Es zeigen:
- Fig. 1: einen Längsschnitt durch die Gasanschlußvorrichtung,
- Fig. 2: eine Ansicht gemäß Pfeil II in Fig. 1,
- Fig. 3: eine Ansicht gemäß Pfeil III in Fig. 1,
- Fig. 4: einen Detailschnitt gemäß der Linie IV-IV in Fig. 1 in vergrößertem Maßstab und
- Fig. 5: eine Detailansicht gemäß Pfeil V in Fig. 1.

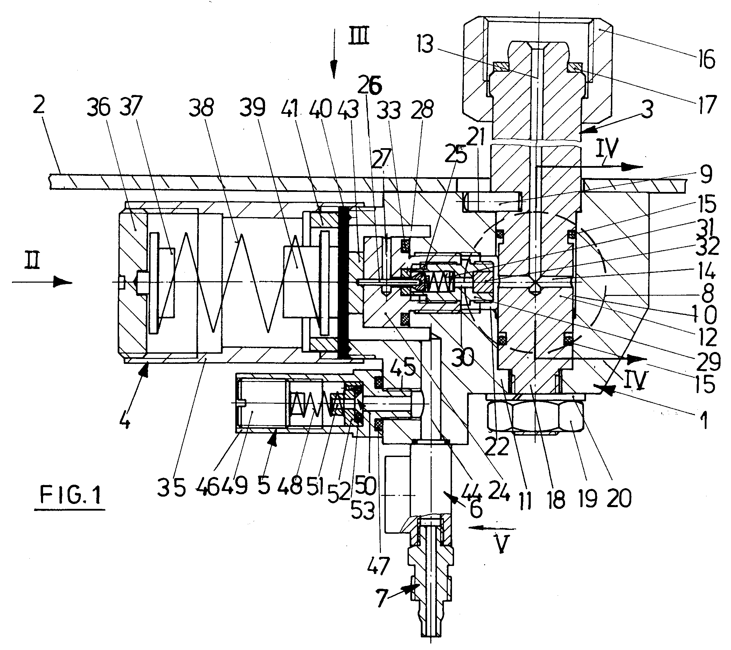

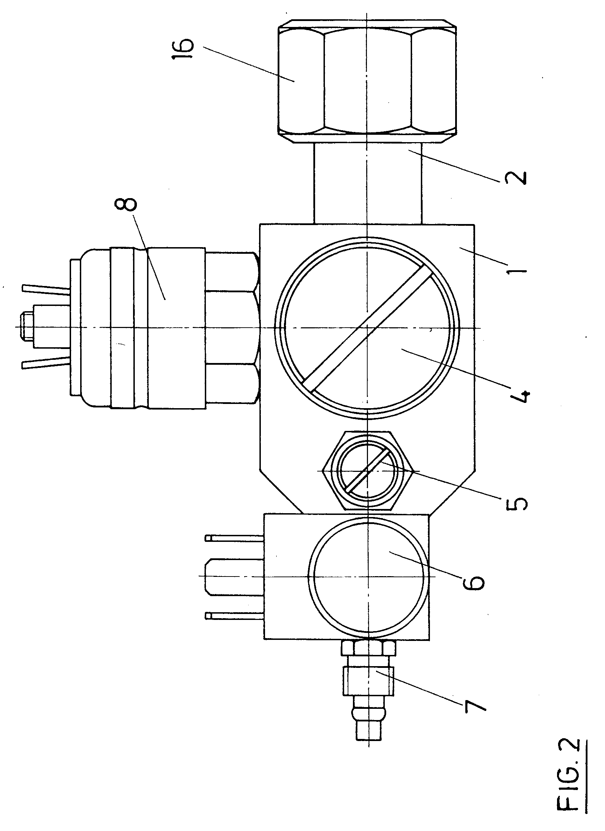

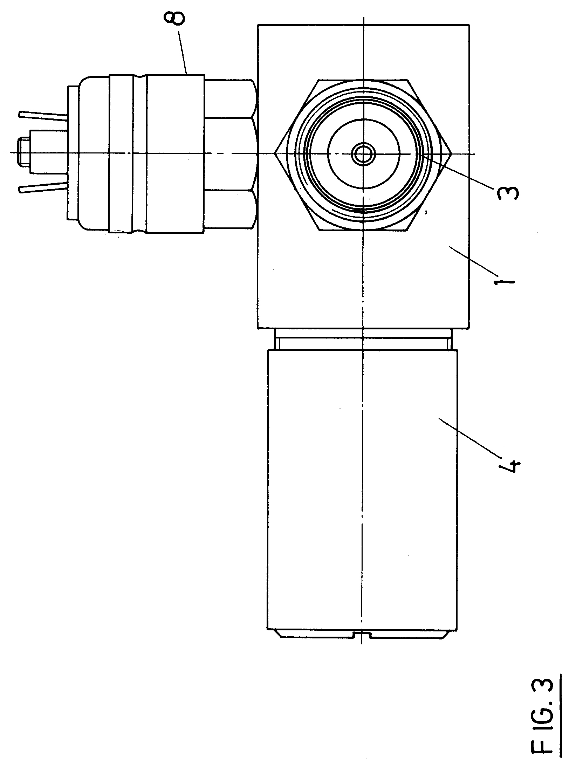

Die Gasanschlußvorrichtung für Insufflationsgeräte umfaßt einen blockförmigen, metallischen Hauptkörper 1, der mit einer Seitenfläche fest mit der Gerätewandung 2 verbunden ist. Mit dem Hauptkörper 1 ist ein Anschlußstutzen 3 für die Gaszufuhr von einer CO₂-Vorratsflasche lösbar und auswechelbar verbunden. Auf der in Fig. 1 links dargestellten Seite des Hauptkörpers 1 befindet sich ein Druckminderer 4, der den hohen Vorratsdruck in der CO₂-Gasflasche von z.B. 70 bar auf den Arbeitsdruck von 2 bar mindert. Neben dem Druckminderer 4 ist ein Sicherheitsventil 5 angeordnet, das der Überdrucksicherung dient. Im rechten Winkel zum Sicherheitsventil 5 befindet sich ein Magnetventil 6 mit daran angeschlossenem Gasauslaßstutzen 7. Senkrecht zu den Achsen des Anschlußstutzens 3 und des Druckminderers 4 ist ein Druckmeßgerät 8 an den Hauptkörper 1 angeschlossen, wie es in den Figuren 2 bis 4 dargestellt ist. Dieses Druckmeßgerät 8 ist als elektronischer Druckwächter ausgebildet.

Zur Aufnahme des Anschlußstutzens 3 ist im Hauptkörper 1 ein erster Kanal 10 als kreiszylindrische Bohrung eingebracht, an deren der Gerätewandung 2 abgewandten Ende ein nach innen ragender umlaufender Bund 11 ausgebildet ist, an welchen der Anschlußstutzen 3 stirnseitig anstößt. Der Anschlußstutzen 3 selbst ist als Zylinderstück 12 ausgebildet und mit einer Sacklochbohrung 13 und einer diese vor dem inneren Ende querenden Durchgangsbohrung 14 sowie vor und hinter deren Mündungen mit am Umfang des Zylinderstückes 12 angeordneten Dichtungen 15 in Form von O-Ringen versehen, die den mittleren Bereich des Hauptkanals 10 nach außen hin abdichten. Das Zylinderstück 12 des Anschlußstutzens 13 ist am äußeren Ende mit Verbindungselementen in Form einer Überwurfmutter 16 und einer Dichtung 17 versehen, um die Verbindung mit der CO₂-Gasvorratsflasche herzustellen. Die so gebildeten Verbindungselemente können als Cartuschen-, Pinn-Index-, amerikanische-CO₂-, deutsche-CO₂-, japanische-CO₂ oder deutsche N₂O- Stutzen ausgebildet sein. Der Anschlußstutzen 3 ist am inneren Ende mit lösbaren Befestigungselementen in Form eines Gewindeansatzes 18, einer auf diese aufgeschraubten Überwurfmutter 19 und einer darunter befindlichen Sicherungsscheibe 20 versehen, wobei der Gewindeansatz 18 die innere Öffnung des Bundes 11 des Hauptkörpers 1 durchgreift und der Anschlußstutzen 3 durch Festschrauben der Überwurfmutter 19 am Hauptkörper 1 festgelegt wird. Durch diese Ausbildung ist der Anschlußstutzen 3 bei im Gehäuse des Insufflationsgerätes montierter Gasanschlußvorrichtung auswechselbar. Zur lagegerechten Anordnung der Durchgangsbohrung 14 des Anschlußstutzens 3 und zugleich zu dessen Verdrehsicherung ist in dessen Zylinderstück 12 ein Zylinderstift 9 radial eingesetzt, dem eine Radialnut 21 im Hauptkörper 1 zugeordnet ist. Die Verdrehsicherung des Anschlußstutzens 9 ist insbesondere beim Pin-Index-Stutzen sehr wichtig, da gleichzeitig eine Positionierung der Gasflasche erfolgt.

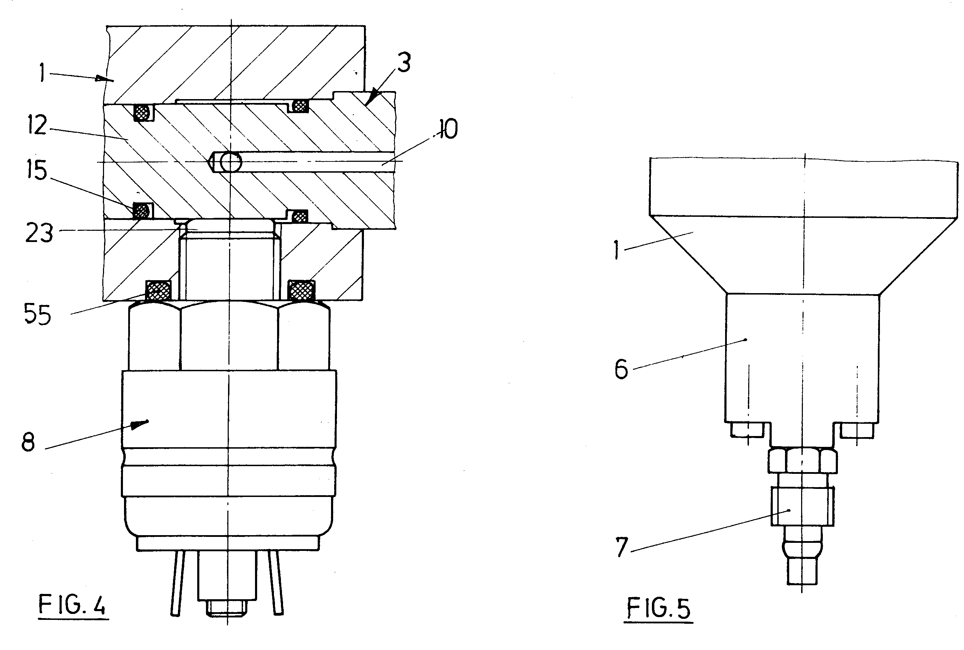

In den Hauptkanal 10 münden ein erster Querkanal 22 ( Fig. 1) zur Aufnahme des Druckminderers 4 und senkrecht dazu ein zweiter Querkanal 23 ( Fig. 4) zur Aufnahme des Druckmeßgerätes 8 in Form eines elektronischen Druckwächters. In den mit einem Innengewinde versehenen Querkanal 22 ist ein Gewindestück 24 eingeschraubt, das mit einer, eine Ventilnadel 25 aufnehmenden Axialbohrung 26 und einer in diese mündenden Radialbohrung 27 versehen ist, die in einen U-förmigen Druckraum 28 mündet. In die zum Hauptkanal 10 gerichtete Vorderseite des Gewindestückes 24 ist ein Druckstück 29 eingeschraubt, das mit einer Axialbohrung 30 versehen ist, auf deren einer Seite eine die Ventilnadel 25 beaufschlagende Druckfeder 31 und auf deren anderen, zum Hauptkanal 10 gerichteten Seite eine Sinterbuchse 32 angeordnet sind. Das Gewindestück 24 ist mit seinem Kopfteil mittels eines O-Ringes 33 abgedichtet in den Hauptkörper 1 eingeschraubt.

Der Druckminderer 4 umfaßt ferner einen auf das Außengewinde eines den Druckraum 28 umgebenden Gewindeansatzes 34 des Hauptkörpers 1 aufgeschraubten Hülsenkörper 35, der am Ende durch einen in ein Innengewinde eingeschraubten und einstellbaren Verschlußstopfen 36 verschlossen ist. Dieser trägt auf seiner Innenseite einen Aufnahmekörper 37 für eine weitere Druckfeder 38, deren anderes Ende auf eine Federführung 39 gelagert ist, die auf einer Membrane 40 aufgesetzt ist, die zwischen einem Ring 41 im Innern des Hülsenkörpers 35 und dem Gewindeansatz 34 des Hauptkörpers 1 eingespannt ist. Auf der zum Hauptkörper 1 gerichteten Seite der Membrane 40 befindet sich eine Scheibe 43, in welche die Ventilnadel 25 eingreift. Der somit durch die einstellbare Federkraft der Druckfeder 38 einstellbare Druckminderer 4 mindert den Hochdruck des über den Anschlußstutzen 3 zugeführten Gasdruckes auf den Arbeitsdruck des Insufflationsgases, welches über den Druckraum 28 einem Auslaßkanal 44 im Hauptkörper 1 zugeführt wird. Dieser mündet in das Magnetventil 6, an welches der Gasauslaßstutzen 7 angeschlossen ist, wie es Fig. 1 zeigt. Unmittelbar vor dem Magnetventil 6 mündet in den Auslaßkanal 44 eine Gewindebohrung 45, in welche das Sicherheitsventil 5 eingesetzt ist. Dieses besteht aus einem zylindrischen Ventilkörper 46, der unter Zwischenlage eines O-Ringes 47 stirnseitig in die Gewindebohrung 45 eingeschraubt ist. Eine Druckfeder 48 erstreckt sich zwischen einer einstellbaren Federdruckschraube 49 und dem Ventilkörper 50, der aus einer inneren Halterung 51 und einer äußeren Halterung 52 für einen O-Ring 53 gebildet ist.

Schließlich ist gemäß Fig. 4 das Druckmeßgerät 8 in Form eines elektronischen Druckwächters mit seinem Gewindeansatz 54 unter Zwischenlage eines O-Ringes 55 in den Querkanal 23 im Hauptkörper 1 eingeschraubt. Der Hauptkanal 10 und die beiden Querkanäle 22, 23 stehen im Bereich zwischen den beiden Dichtungen 15 des Anschlußstutzens 3 miteinander druckmäßig in Verbindung.

| **BEZUGSZEICHENLISTE** | | | |
|---|---|---|---|
| 1 | Hauptkörper | 25 | Ventilnadel |
| 2 | Gerätewandung | 26 | Axialbohrung |
| 3 | Anschlußstutzen | 27 | Radialbohrung |
| 4 | Druckminderer | 28 | Druckraum |
| 5 | Sicherheitsventil | 29 | Druckstück |
| 6 | Magnetventil | 30 | Axialbohrung |
| 7 | Gasauslaßstutzen | 31 | Druckfeder |
| 8 | Druckanzeigegerät | 32 | Sinterbuchse |
| 9 | Zylinderstift | 33 | O-Ring |
| 10 | Hauptkanal | 34 | Gewindeansatz |
| 11 | Bund | 35 | Hülsenkörper |
| 12 | Zylinderstück | 36 | Verschlußstopfen |
| 13 | Sacklochbohrung | 37 | Aufnahmekörper |
| 14 | Durchgangsbohrung | 38 | Druckfeder |
| 15 | Dichtung | 39 | Federführung |
| 16 | Überwurfmutter | 40 | Membrane |
| 17 | Dichtung | 41 | Ring |
| 18 | Gewindeansatz | 42 | |
| 19 | Überwurfmutter | 43 | Scheibe |
| 20 | Scheibe | 44 | Auslaßkanal |
| 21 | Radialnut | 45 | Gewindebohrung |
| 22 | Querkanal | 46 | Ventilkörper |
| 23 | Querkanal | 47 | O-Ring |
| 24 | Gewindestück | 48 | Druckfeder |
| 49 | Federdruckschraube | | |
| 50 | Ventilkörper | 53 | O-Ring |
| 51 | innere Halterung | 54 | Gewindeansatz |
| 52 | äußere Halterung | 55 | O-Ring |

## Patentansprüche

1. Gasanschlußvorrichtung für Insufflationsgeräte, mit einem Anschlußstutzen für die Gaszufuhr, mit einem Druckminderer, mit einem Sicherheitsventil und mit einem, mit einem Magnetventil versehenen Gasauslaßstutzen,
**dadurch gekennzeichnet,**
daß der Anschlußstutzen (3), der Druckminderer (4), das Sicherheitsventil (5) und das Magnetventil (6) mit Gasauslaßstutzen (7) an einen gemeinsamen Hauptkörper (1) angeschlossen und durch im Hauptkörper (1) ausgebildete Kanäle (10,22,23,44) miteinander verbunden sind.

2. Gasanschlußvorrichtung nach Anspruch 1, dadurch gekennzeichnet, daß der Anschlußstutzen (3) lösbar und auswechselbar im Hauptkörper (1) angeordnet ist.

3. Gasanschlußvorrichtung nach Anspruch 1 oder 2, dadurch gekennzeichnet, daß der Hauptkörper (1) zur lösbaren Aufnahme des Anschlußstutzens (3) einen Hauptkanal (10), einen in diesen mündenden, zur Aufnahme des Druckminderers (4) dienenden Querkanal (22) und einen in diesen mündenden Auslaßkanal (44) aufweist, an den das Sicherheitsventil (5) und der Gasauslaßstutzen (7) mit vorgeschaltetem Magnetventil (6) angeschlossen sind.

4. Gasanschlußvorrichtung nach Anspruch 3, dadurch gekennzeichnet, daß quer zum Hauptkanal (1) ein in diesen mündender weiterer Querkanal (23) eingebracht ist, in den ein Druckanzeigegerät (8) eingesetzt ist.

5. Gasanschlußvorrichtung nach einem der Ansprüche 1 bis 4, dadurch gekennzeichnet, daß der Anschlußstutzen (3) als Zylinderstück (12) ausgebildet und mit einer Sacklochbohrung (13) und einer diese vor dem inneren Ende querenden Durchgangsbohrung (14) sowie vor und hinter deren Mündungen am Umfang des Zylinderstückes (12) angeordneten Dichtungen (15) versehen ist, die den Bereich des Hauptkanales (10) und den Bereich der in diesen mündenden Querkanäle (22,23) für den Druckminderer (5) und das Druckanzeigegerät (8) nach außen abdichten, und daß der Anschlußstutzen (3) am äußeren Ende mit Verbindungselementen zum Anschluß einer Gasvorratsflasche und am inneren Ende mit lösbaren Befestigungselementen versehen ist.

6. Gasanschlußvorrichtung nach Anspruch 5, dadurch gekennzeichnet, daß die lösbaren Befestigungselemente aus einem Gewindeansatz (18) des Zylinderstückes (12) und einer aufgeschraubten Überwurfmutter (19) gebildet sind, welche einen am inneren Ende des Hauptkanales (10) ausgebildeten, nach innen ragenden, umlaufenden Bund (11) des Hauptkörpers (1) zwischen sich einspannen.

7. Gasanschlußvorrichtung nach einem der vorangegangenen Ansprüche, dadurch gekennzeichnet, daß ein Zylinderstift (9) radial in eine Sacklochbohrung des Zylinderstückes (12) eingebracht ist und daß eine Radialnut (21) am Umfang des Hauptkanales (10) des Hauptkörpers (1) zur positionsgenauen Aufnahme des Anschlußstutzens (3) und zu dessen Verdrehsicherung im Hauptkörper (1) vorgesehen ist.

8. Gasanschlußvorrichtung nach einem der Ansprüche 1 bis 7, dadurch gekennzeichnet, daß der Druckminderer (4) aus einem in das Innengewinde des Querkanales (22) eingeschraubten Gewindestück (24), das mit einer eine Ventilnadel (25) aufnehmenden Axialbohrung (26) und einer in diese mündenden Radialbohrung (27) versehen ist, und aus einem in dessen Vorderseite eingeschraubten Druckstück (29) gebildet ist, das mit einer Axialbohrung (30) und einer in dieser angeordneten, die Ventilnadel (25) beaufschlagenden Druckfeder (31) versehen ist, und daß das Druckstück (29) am Eingang der Axialbohrung (26) mit einer Sinterbuchse (30) versehen ist.

9. Gasanschlußvorrichtung nach Anspruch 8, dadurch gekennzeichnet, daß die Ventilnadel (25) von einer unter einstellbarem Federdruck stehenden Membrane (40) beaufschlagt ist.

## Geänderte Patentansprüche

### Geänderte Patentansprüche gemäss Regel 86(2) EPÜ.

1. Gasanschlußvorrichtung für Insufflationsgeräte, mit einem Anschlußstutzen für die Gaszufuhr, mit einem Druckminderer, mit einem Sicherheitsventil und mit einem, mit einem Magnetventil versehenen Gasauslaßstutzen,
**dadurch gekennzeichnet,**
daß ein Hauptkörper (1) einen Hauptkanal (10) zur lösbaren Aufnahme des Anschlußstutzens (3), einen in diesen mündenden, zur Aufnahme des Druckminderers (4) dienenden Querkanal (22) und einen in diesen mündenden Auslaßkanal (44) aufweist, an den das Sicherheitsventil (5) und der Gasauslaßstutzen (7) mit vorgeschaltetem Magnetventil (6) angeschlossen sind, und daß quer zum Hauptkanal (10) ein in diesen mündender weiterer Querkanal (23) eingebracht ist, in den das Druckanzeigegerät (8) eingesetzt ist.

2. Gasanschlußvorrichtung nach Anspruch 1, dadurch gekennzeichnet, daß der Anschlußstutzen (3) als Zylinderstück (12) ausgebildet und mit einer Sacklochbohrung (13) und einer diese vor dem inneren Ende querenden Durchgangsbohrung (14) sowie vor und hinter deren Mündungen am Umfang des Zylinderstückes (12) angeordneten Dichtungen (15) versehen ist, die den Bereich des Hauptkanales (10) und den Bereich der in diesen mündenden Querkanäle (22,23) für den Druckminderer (5) und das Druckanzeigegerät (8) nach außen abdichten, und daß der Anschlußstutzen (3) am äußeren Ende mit Verbindungselementen zum Anschluß einer Gasvorratsflasche und am inneren Ende mit lösbaren Befestigungselementen versehen ist.

3. Gasanschlußvorrichtung nach Anspruch 2, dadurch gekennzeichnet, daß die lösbaren Befestigungselemente aus einem Gewindeansatz (18) des Zylinderstückes (12) und einer aufgeschraubten Überwurfmutter (19) gebildet sind, welche einen am inneren Ende des Hauptkanales (10) ausgebildeten, nach innen ragenden, umlaufenden Bund (11) des Hauptkörpers (1) zwischen sich einspannen.

4. Gasanschlußvorrichtung nach einem der vorangegangenen Ansprüche 2 oder 3, dadurch gekennzeichnet, daß ein Zylinderstift (9) radial in eine Sacklochbohrung des Zylinderstückes (12) eingebracht ist und daß eine Radialnut (21) am Umfang des Hauptkanales (10) des Hauptkörpers (1) zur positionsgenauen Aufnahme des Anschlußstutzens (3) und zu dessen Verdrehsicherung im Hauptkörper (1) vorgesehen ist.

5. Gasanschlußvorrichtung nach einem der Ansprüche 1 bis 4, dadurch gekennzeichnet, daß der Druckminderer (4) aus einem in das Innengewinde des Querkanales (22) eingeschraubten GewindeStück (24), das mit einer eine Ventilnadel (25) aufnehmenden Axialbohrung (26) und einer in diese mündenden Radialbohrung (27) versehen ist, und aus einem in dessen Vorderseite eingeschraubten Druckstück (29) gebildet ist, das mit einer Axialbohrung (30) und einer in dieser angeordneten, die Ventilnadel (25) beaufschlagenden Druckfeder (31) versehen ist, und daß das Druckstück (29) am Eingang der Axialbohrung (26) mit einer Sinterbuchse (30) versehen ist.

6. Gasanschlußvorrichtung nach Anspruch 5, dadurch gekennzeichnet, daß die Ventilnadel (25) von einer unter einstellbarem Federdruck stehenden Membrane (40) beaufschlagt ist.
